# EUROPEAN PATENT APPLICATION

(11) **EP 3 098 738 A1**
(43) Date of publication of application: **30.11.2016**
(21) Application number: 15169894.1
(22) Date of filing: 29.05.2015
(51) Int. Cl.: G06F 19/00

(54) **AEROSOL NEBULIZER CONTROL DEVICE**

(71) Applicant: PARI Pharma GmbH, 82319 Starnberg (DE)
(72) Inventor: Finke, Matthias, 81475 München (DE); Achtzehner, Wolfgang, 82239 Alling (DE)
(74) Representative: Hoffmann Eitle

(57) **Abstract**

The present invention relates to a control device for an aerosol nebulizer, comprising: a communication unit (100), configured to establish a wireless communication connection and to perform data communication with a communication device (20); a control unit (200), configured to control an operation of the aerosol generator; a storage unit (300), configured to store in advance an identification value of said communication device (20), wherein, based on said identification value, said communication unit (100) is further configured to establish said wireless communication connection by an authentication of the communication device (20) based on the identification value of the communication device (20).

## Description

### TECHNICAL FIELD

The present invention relates to an aerosol nebulizer control device and a system comprising an aerosol nebulizer control device and an aerosol nebulizer.

### BACKGROUND

Aerosol nebulizers are known to have an aerosol generator to generate an aerosol from a liquid medication. To improve the usability of the aerosol nebulizers for the user, an external control device may be used. Such a control device may comprise a processor, a memory, for example in the form of a nonvolatile memory, a display, and an input unit which may be configured in the form of a keyboard, individual buttons, or a touch-sensitive surface in the display. Such an external control device may additionally operate to establish wireless communication connections with the aerosol nebulizer to transfer configuration data to the aerosol nebulizers to appropriately set and control the operation of the aerosol generator. Similarly, nebulization data and/or measurement data may be generated and transferred from the aerosol nebulizers to the external control device. Such nebulization data and/or measurement data may, for example, be generated by an internal control unit of the aerosol nebulizers or sensors that are mounted at the aerosol nebulizers. An example of such an external control device is an external computing device, for example in the form of a smartphone or a PDA as described in DE 102 43 371 A1 or US 2006/0237001 A1. Such an external control device may further exhibit a telecommunication module and may thus further offer the capability to transfer the control data and inhalation/measurement data via the internet to a central data base, for example for telemedicine purposes and for the purpose of (centralized) electronic health records.

However, the proper setup and operation of the wireless communication connection between the aerosol nebulizer and the external control device still requires a manual input from the user, for example in the form of inputting specific connection data or the like, and is therefore disadvantageous because a user/patient, which is potentially in need of an urgent inhalation due to medical reasons, does not want to be burdened with the setup and the proper and reliable operation of the wireless communication connection.

In addition, users/patients may be very sensitive as to the transfer of personal health care data, including the above inhalation/measurement data, via an unknown and insecure data channel. As such, confidentiality and integrity of personal health data are key attributes that are not guaranteed when conventionally transferring data between an aerosol nebulizer to a central server.

Against this background, it is an objective of the present invention to provide an aerosol nebulizer control device that overcomes the above disadvantages of manual user inputs for establishing a wireless communication connection and guarantees personal health care data security.

### SUMMARY

The features of an aerosol nebulizer control device according to the present description are defined in claim 1. Advantageous embodiments are described in the dependent claims.

### BRIEF DESCRIPTION OF THE DRAWINGS

**Fig. 1** shows a schematic illustration of a control device for an aerosol nebulizer according to an embodiment.
**Fig. 2** shows a schematic illustration of a control device for an aerosol nebulizer according to another embodiment.
**Fig. 3** shows a schematic flow chart according to an embodiment.
**Fig. 4** shows a schematic illustration of an operation flow according to another embodiment.
**Fig. 5** shows a schematic illustration of an operation flow according to another embodiment.
**Fig. 6** shows a schematic illustration of an operation flow according to another embodiment.

### DESCRIPTION OF THE EMBODIMENTS

Embodiments of the present invention are described with reference to the Figures. It is noted that the following description should not be construed as limiting the invention. In the following, similar or same reference signs indicate similar or same elements or operations.

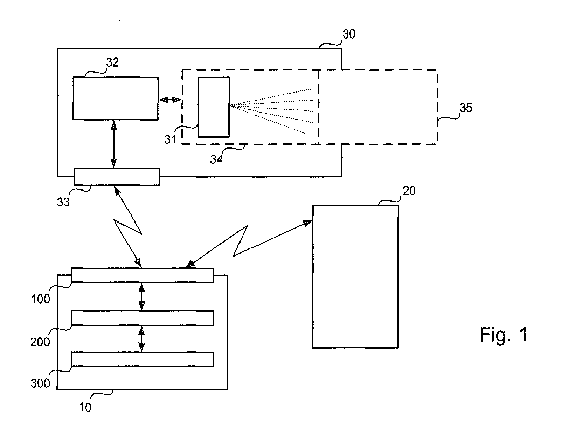

**Fig. 1** shows an aerosol nebulizer control device 10 according to an embodiment in combination and communication interaction with a communication device 20 and an aerosol nebulizer 30.

The control device 10 is in communication interaction (wireless or wire based, as well as electronically connected or directly integrated) with an aerosol nebulizer 30 that may comprise an aerosol generator 31, for example a membrane aerosol generator or the like, that is suitable to nebulize a liquid medication, which is held in a reservoir (not shown) of the aerosol nebulizer 30. The liquid medication may be a fluid that contains at least one medical drug. The liquid medication is transformed into an aerosol by the aerosol generator 31, which subsequently enters an inhalation chamber 34 that is connected to a mouthpiece 35. From the mouthpiece, the patient/user inhales the aerosol into the respiratory tract, like lung, throat, nose or sinuses, to perform a medical treatment according to an aerosol therapy protocol. Further, the aerosol generator 31 operates according to configuration data that may be provided from the control device 10 via a communication interface 33 and an operation unit 32 of the aerosol nebulizer 30, and the aerosol nebulizer 30 provides inhalation data and/or measurement data generated by one or more sensors (not shown) at the aerosol nebulizer 30 via the communication interaction to the control device 10 alternatively the communication is performed by an communication interface. Examples of such sensors are sensors for detecting a state of a membrane aerosol generator, for detecting a filling level of a liquid medication in a reservoir of the aerosol nebulizer 30, for detecting inhalation/exhalation flow/volume/time characteristics during the use of the aerosol nebulizer 30 of the user/patient, and the like.

As shown in **Fig. 1****,** the control device 10 is provided separately from the aerosol nebulizer 30 in order to separate the control functionality as much as possible from aerosol nebulizer 30. This is, however, not considered to be limiting and the control device 10 may alternatively be provided as an integral part of the aerosol nebulizer 30, for example in the form of an integral unit of the aerosol nebulizer, for example as a suitably adapted operation unit 32.

As further shown in **Fig. 1****,** the control device 10 comprises a communication unit 100, a control unit 200, and a storage unit 300.

The communication unit 100 of the control device 10 may establish a wireless communication connection with the separate communication device 20 and may perform data communication with the communication device 20 via the established wireless communication connection. The established wireless communication connection may be a Bluetooth connection, a near field connection, a WiFi connection or the like. Via the established wireless communication connection a data communication between the communication unit 100 of the control device 10 and the separate communication device 20 may be performed so that the communication unit 100 may send/receive data to/from the external communication device 20. As will be further described below such a data transfer may comprise, for example, the transfer of configuration data for the proper operation of the control device 10 and/or the aerosol generator as well as the transfer of measurement data and/or inhalation data. As will also be described below, the data communication may be performed in a secure and reliable way.

The communication device 20 may be a customized communication hub, a portable device such as a smartphone or a tablet having a customized application program, or the like. The communication device 20 may also be a customized communication hub without an input unit and/or a display unit and may be activated and/or deactivated or provided with information as to the establishment of the wireless communication connection or the operation data for the aerosol nebulizer 30 from a central server (not shown). The communication device 20 may thus itself be connected, for example via a cloud server, with a central server which may be a medical evaluation device or the like to receive and evaluate the above measurement data and/or inhalation data, and to provide a feedback to the control device 10 so that the inhalation therapy and/or the operation of the aerosol generator is suitably adjusted.

In an alternative embodiment the communication device 20 may itself evaluate the above measurement data and/or inhalation data, and provide a feedback to the control device so that the aerosol therapy and/or the operation of the aerosol generator is suitably adjusted.

In another alternative embodiment, the wireless communication connection between the control device 10 and the communication device 20 or the central server may be established by radio communication, for example by providing the control device 10 with a radio communication module, such as a GSM module, UMTS module, LTE module, with or without a SIM card, or the like.

The control unit 200 may control an operation of the aerosol generator, for example, based on the received configuration data. Different operation modes of the control device 10 may thus be selected, as will be further described below.

The storage unit 300, which may be an internal EEPROM or the like, may store *in advance* an identification value of the communication device 20. That is, the identification value of the communication device 20 may be stored during the production stage of the control device, so that the control device 10 is already provided with the identification value when purchasing the control device 10 and thus before the first establishment of the wireless communication connection when the user/patient uses the control device for the first time in the patient environment. Alternatively, the identification value of the communication device 20 may be stored in the storage unit 300 before the first establishment of the wireless communication connection after purchasing the control device 10. This means that the control device 10 is limited from the beginning to establish a wireless communication connection with a communication device that is known *in advance* via the stored identification value. If the wireless communication connection is, for example, a Bluetooth connection, then the corresponding identification value may be the Bluetooth friendly NAME and/or a Bluetooth-PIN-Code being provided to the communication device 20 and being stored in advance in the storage unit 300 of the control device 10. In addition, the storage unit 300 may also store the received configuration data and/or the inhalation/measurement data until a successful data transfer is possible by the data communication to the communication device 20.

Further, in order to allow establishment of the above wireless communication connection, the communication unit 100 may authenticate the communication device 20 based on the identification value stored *in advance* in the storage unit 300 of the control device 10. That is, the communication unit 100 may determine or confirm the identity of the external communication device 20 before attempting to establish the wireless communication connection. Based on this authentication, the establishment of a communication connection is restricted to an external communication device that is known *in advance* based on the stored identification value. In other words, the communication device 20 is reserved or preset *in advance* for data communication with said communication unit 100, and the control device 10 may establish a wireless communication connection only with the communication device 20 that is known *in advance* based in the stored identification value. As such, the user is not burdened with performing an authentication process, no passkeys or the like are required, and the control device 10 does not have to scan for other devices. The wireless communication connection may thus be reliably established. Further, the data communication may be performed within a predefined network, which does not allow third parties to gain access to the configuration data and/or the inhalation/measurement data.

In a further embodiment, the above identification value may not be a unique identification of the communication device 20, but may identify a specific group of communication devices, for example of the same manufacturer, having the same model number, or the like. For example, a Bluetooth friendly NAME may be used as the identification value so that a customized communication hub as communication device 20 may easily be replaced with another customized communication hub of the same type/model (for example, when a communication hub is lost, is not working properly, or the like).

In a further embodiment, the above authentication may be performed in the communication unit 100 by comparing the identification value of the communication device 20 which is stored in the storage unit 300 with an identification value that is received from the communication device 20 when the establishment of the wireless communication connection is started *for the first time,* for example after purchasing the control device 10 or the patient uses the control device 10 for the first time.

An example of this initial establishment of the wireless communication connection is an initial pairing process between the control device 10 and the communication device 20, such as when the control device 10 or the communication unit 100 is a Bluetooth master device and performs an enquiry scan and the communication device 20 responds by transmitting an identification value, for example, the MAC address, the Bluetooth friendly NAME and/or the Bluetooth PIN.

If the communication unit 100 determines that the stored identification value matches with the received identification value, it initiates a pairing process to establish said wireless communication connection by transmitting a pairing message. This pairing message includes an identification value of the control device 10, for example the MAC address of the control device 10 and/or the serial number of the control device 10. When receiving the pairing message, the communication device performs a determination as to whether the received identification value of the control device 10 is stored in a white list in association with at least one identification of the communication device 20, for example in the form of an association table or the like. The identification of the communication device 20 may include the MAC address, a (Bluetooth friendly) NAME, and/or a serial number of the communication device 20, and the association in the white list defines a *valid* combination of a communication unit 100 and the communication device 20 which are thus allowed to establish the wireless communication connection. This valid combination of identification values may be provided or canceled from a central server to the communication device 20 that may thus activate or deactivate the establishment of the wireless communication connection and also perform an evaluation of measurement and/or inhalation data that are provided from the control device 10.

If the transmitted identification value of the control device 10, which is included in the above pairing message, matches an identification value of the control device 10 that is stored *in advance in association* with the above identification of the communication device, i.e. if the combination of the control device 10 and the communication device 20 is a valid combination in the above white list, then the communication device 20 transmits a pairing acceptance message which is received at the communication unit 100 of the control device 10. As a result, a wireless communication connection may successfully be established, in particular without a manual setting of a user after purchasing the control device/aerosol nebulizer. This is advantageous for patients/users that are not, for example, Bluetooth-savvy or are in such a medical state in which they do not wish to be additionally burdened with setting up a wireless communication connection. If, on the other hand, the combination of the received control device 10 identification value and the communication device 20 identification is not a valid combination on the above white list, then the communication device 20 may not respond at all.

In another embodiment, the communication unit 100 receives configuration data from the communication device 20 via the established wireless communication connection. The configuration data may be data for operating the aerosol nebulizer 30 and/or data for operating the control device 10, and are transferred from the communication unit 100 to the control unit 200.

In an embodiment, the configuration data may comprise at least one of a current date, a current time, a maximum nebulization duration, a number of maximal storable records, an automatic data transfer deactivation, and an identification of one or more drugs usable for said liquid medication or so called aerosol therapy or inhalation therapy. The provision of the current time and date may be provided in order to synchronize in time the operation of the control device and/or the operation of the aerosol nebulizer 30 with an external server device that also receives and evaluates inhalation/measurement data. The maximum nebulization duration which may be drug-specific defines a maximum amount of time that the aerosol generator is allowed to generate aerosol from the liquid medication, and may thus be used by the control unit 200 to suitably switch-off the aerosol generator, and thus to cancel a contradicting command from the user. The user may be informed about such a switch-off due to reaching the maximum nebulization duration on an operation unit (which will be described below) of the control device. Further, the automatic data transfer deactivation may indicate to the control device to deactivate inhalation/measurement data transfer to the communication device 20 at the end of an aerosol therapy/inhalation session. Further, the identification of one or more drugs usable for said liquid medication may be notified to the user (via the operation unit to be described below). Further, the number of maximal storable records indicates a maximal number of data records or data sets that are storable in the control device 10. A data record or a data set comprises, for example, all data being related to a single session of the aerosol therapy protocol. Here, the number of maximal storable records is usually fixed, but may be changed according to software and/or hardware updates. The number of maximal storable records may be used in the control device to timely issue a visual and/or audible warning to the user to indicate that a wireless communication connection should be established to transfer the stored data records. For example, such a warning may be issued when 90 data records of respective sessions of the aerosol therapy protocol have been stored and the number of maximal storable records is 100.

In a further embodiment, the communication unit 100 may further operate to determine whether the configuration data are correctly received via the established wireless communication connection. For example, the configuration data may be transmitted including controlling bytes, such as a check sum, a hash sum, or the like. The communication unit 100 may thus perform a cyclic redundancy check (CRC) for the received configuration data. In addition, the communication unit 100 may perform a format verification as to whether the received configuration data are conform with a format that is required by the control unit 200. Further, if the communication unit 100 determines that configuration data have been correctly transmitted, for example based on the fact that the received check sum matches a calculated check sum over the received configuration data, then the communication unit 100 sends an acknowledgement message, for example a (positive) acknowledgement (ACK) protocol message, to the communication device 20. In the case that the configuration data have not been correctly transmitted, the communication unit 100 sends a negative acknowledgment (NAK) protocol message to the communication device 20 so that the configuration data may be sent again, for example the next time the communication unit 100 connects with the communication device 20.

In a further embodiment, the communication unit 100 may transmit inhalation data and/or measurement data to the communication device 20. The inhalation data may, for example, be included in a payload of a communication message that is used to perform the data communication. The communication unit 100 may further enclose the inhalation data and/or measurement data with controlling bytes, for example by concatenating or linking the inhalation data with a check sum or the like, as described above, so that the communication device 20 (or, alternatively, a cloud server, a central server or a central unit within an IT network) may determine whether the inhalation data have been transmitted correctly. This communication message may include an identification value of the control device 10, but preferably does not include personal information so as to identify the user/patient.

For example, the inhalation data may include at least one of a nebulization date, a nebulization time, a nebulization duration, a communication time of the control device 10, a reason for a switch-off of the aerosol nebulizer 30, a usage status by a user, and a list of one or more drugs used for said liquid medication. The inhalation data may, for example, be generated by the control unit 200 that monitors the operation of the aerosol generator and the aerosol nebulizer 30. Here, the reason for a switch-off may be a manual switch off by the user, a forced switch-off due to reaching the maximum nebulization duration, absence of a suitable amount of liquid medication, or the like. Here, pre-determined reasons for switch-off may be provided at the control unit 200 of the control device 10, like i.e. time, volume or liquid level in the fluid reservoir as well as a user contact or inhalation maneuver (flow, volume or time characteristics of inhalation/exhalation) of the user. Alternatively, the user may input a reason for switch-off via a later described operating unit of the control device.

For example the usage status by a user of the aerosol nebulizer 30 may be detected by a sensor (not shown) included in the aerosol nebulizer 30. The sensor (not shown) may be included in or attached to the mouthpiece, mixing chamber, inhalation/exhalation valves, and/or aerosol generator. The sensor sends a signal via the communication to the communication device 10 or communication unit 100 to evaluate whether a user/patient actually uses the device. For example, the user/patient during the aerosol therapy is in contact with the mouthpiece (contact sensor), inhales through the mixing chamber or on the aerosol mouthpiece (flow sensor or density sensor), or generates an inhalation flow through the device (flow sensor) and thus one or more corresponding sensor signals are generated, which can be communicated and evaluated.

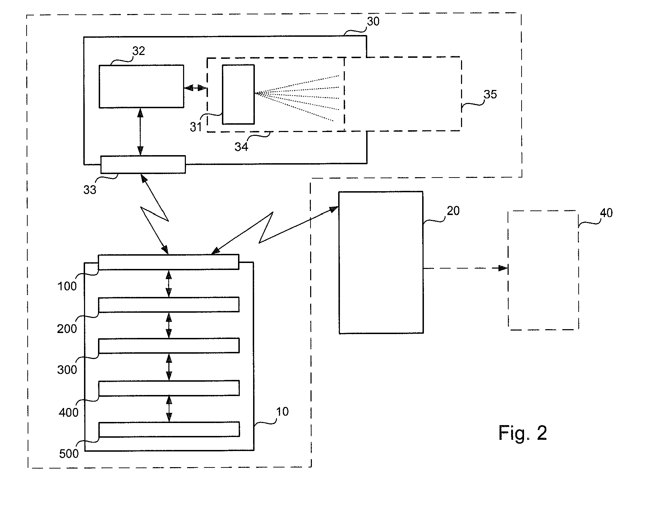

**Fig. 2** shows another embodiment of a control device 10 for an aerosol nebulizer 30. In addition to the units described above with regard to Fig. 1, the control device 10 according to **Fig. 2** further includes an encryption unit 400 and/or an operation unit 500. As shown, the respective units of the control device 10 are in mutual interaction.

The encryption unit 400 may operate to encrypt data used for the data communication via the established wireless communication connection. For example, the encryption unit 400 may encrypt the inhalation data described above. Preferably, the encryption unit 400 may perform the encryption to encode the inhalation data by using an encryption key that is *unknown* to the communication device 20. As such, the communication device 20 may not be able to decrypt the inhalation data, but may operate to transfer the encrypted inhalation data further to a central device 40, such as a cloud server, a central server, a web portal, a central unit of an IT network, based, for example, on an intranet or internet. When using a message for transferring the encrypted inhalation data further to the central server device for aerosol therapy evaluation and/or therapy feedback purposes, for example via a cloud server by radio emissions, the communication device 20 further includes at least an identification value of the control device 10 and an identification value of the communication device 20 in that message. For example, the message may include a serial number of the control device 10 and a serial number of the communication device 20 which are both unique device identification values. Importantly, and in addition to the encryption protection, this message does not include any personal information of the user that may be used by unauthorized third parties to relate the inhalation data or measurement data to an individual person. Instead, the central server may perform a matching of the white list identification value association, described above for the communication device 20, now, for example, at a database at the central server to reliably determine the source of the inhalation data, retrieve an encryption key used at the encryption unit 400, and to subsequently decrypt the encrypted inhalation data. The inhalation data may thus be securely and reliably transferred from a control device 10 of an aerosol nebulizer 30 via an external communication device to a central server for evaluation and feedback purposes in a way in which an intermediate communication device is not able to decrypt or decode the encrypted inhalation data for the purpose of evaluating by an internal processing, no personalized inhalation data are provided to third parties other than the above central server, and the like, see above.

Alternatively, the communication device 20 and the above central server may be part of a virtual private network so that the data communication between the communication unit 100 and the communication device 20 or between the communication unit 100 and the central server may be performed via a secure data channel.

In another embodiment, the control device 10 may be set as a master device when the wireless communication connection is established with the communication device 20. If the wireless communication connection is, for example, a Bluetooth connection, then the control device 10 or the communication unit 100 may be the Bluetooth master device. As such, it is the communication unit 100 that controls, and in particularly initiates, the establishment of the wireless communication connection. Based on this master device setting, the control device 10 may not be discoverable by the communication device 20.

Based on the master device setting the communication unit 100 may further be configured to not attempt a re-establishment of the wireless communication connection with the communication device 20, if the wireless connection is lost, for example because the control device 10 becomes out of reach of the communication device 20. The above inhalation data and/or measurement data may thus be transferred the next time the control device 10 initiates the data transfer process, for example, when the therapy session is finished, when the data transfer process is triggered by the user, or the like.

In another embodiment the communication unit 100 may further transfer the inhalation data and/or measurement data to the communication device 20 at an end of a therapy session. This data transfer may be independent as to whether the aerosol therapy session was properly ended, interrupted by the user via an active switch-off of the control device 10, or accidentally ended. Alternatively, this data transfer may be initialized manually by the user, for example via a (later described) operating unit of the control device 10. Such a data transfer may be deactivated by the user. The technical purpose of this automatic data transfer deactivation may become apparent from a situation in which the aerosol nebulizer 30 is used without an established wireless communication connection. In order to not bother the user with failed data transfer notifications, this automatic data transfer may be deactivated and is instead only manually initiated.

In a further embodiment, the communication unit 100 of the control device 10 may further receive an acknowledgement message from the communication device 20 if the inhalation data and/or measurement data were correctly transmitted/ transferred to the communication device 20. This may be achieved by enclosing the inhalation data and/or measurement data by controlling bytes, as described above, and by the communication device 20 performing, for example, a cyclic redundancy check (CRC) to determine whether the data transmission was successful or not. If this positive acknowledgement message (ACK) is not received by the communication unit 100, then a re-transmission may be performed by the communication unit.

In addition, when it is determined that the data transmission was successful, then the inhalation data and/or measurement data being related to the last therapy session may be deleted from the storage unit 300 of the control device 10. Further, if the data transmission was not or remains not successful, for example, because the wireless communication connection is lost, then the inhalation data and/or measurement data being related to the last therapy session are stored in the storage unit 300 in association with an indication of the therapy session, for example, a therapy session date and time, a therapy session number, or the like.

The operation unit 500 shown in **Fig. 2** may be arranged with at least one button, for example in the form of touch-sensitive button, LED, touch display and or display or the like, for inputting user commands. This operation unit may thus be used by the user to select and/or confirm a liquid drug that is to be used in the aerosol nebulizer 30 when nebulizing the liquid medication with the aerosol generator. For example, based on the provided configuration data described above, the operation unit may provide the user with a list of usable drugs and prompts the user to select and/or confirm a particular drug in connection with the configuration data for a therapy session. For example, the usable drugs may be selected dependent on a user group, like i.e. asthma patients or cystic fibrosis patients. Usable drugs for the group of asthma patients are, for example, a beta sympathomimetic and a corticoid, like for example salbutamol and budesonide, as well as LABA, or LAMA. Usable drugs for the group of cystic fibrosis patients may be, for example, different antibiotics (for example tobramycin), expectorants and/or mucolytics. The usable drugs may be, for example, drugs in clinical trials, alternatively identified as "drug 1", "drug 2" and so on as well as "Drug A" and "Drug B" and so on. Based on the selected and/or confirmed drug, the control unit 200 may determine a particular one of a set of configuration data to be used when operating the aerosol generator to nebulize the selected and/or confirmed drug. For example, the maximum nebulization/inhalation time may be drug-dependent and may thus be set accordingly. A notification message that indicates the drug selected by the operation unit 400 may also be transmitted via the established wireless communication connection to the communication device 20, preferably encrypted by the above encryption unit 400, to subsequently be transferred to the central server for the purpose of therapy session evaluation and therapy session feedback.

In an embodiment, the operation unit 500 may further be used by a user to select an operation mode of the control device 10. The selectable operation mode may refer to a nebulizing mode, a cleaning mode, or a data transfer mode. In the nebulizing mode, the control unit 200 operates to control the operation of the aerosol generator based on the received configuration data, and preferably according to the selected drug. In the cleaning mode, control unit 200 may, for example, operate to clean the aerosol generator, that may e.g. depend on the time regime of the therapy sessions or be determined based on the cumulative amount of nebulized fluid, or drug formulation. Further, in the data transfer mode, the communication unit 100 operates to perform the data communication via the established communication connection so as to receive proper or updated configuration data and to send the nebulization data and/or measurement data.

In another embodiment, the operation unit 500 may further be used by the user to monitor an adherence to a preset therapy protocol. As described above, the aerosol therapy protocol may be based on the received configuration data, for example setting a maximal nebulization/inhalation duration, setting a number of nebulizations/inhalations for a specific date and/or time, and the like. As further described, such a therapy protocol may be drug specific, and the operation unit may thus indicate a drug-specific adherence to an aerosol therapy protocol and/or to monitor adherence of the user to a preset therapy. This may be achieved by an internal evaluation of inhalation data and/or measurement data generated by the control unit 200 and/or sensors provided at the aerosol nebulizer 30, in particular at the aerosol generator, at the reservoir for holding the liquid medication and supplying it to the aerosol generator, or the like. As such, if the nebulization of the selected drug occurs in agreement with the preset therapy protocol, then the operation unit provides a corresponding visual and/or audible indication. For example, the operation unit may be provided with a display to indicate that the user follows the therapy protocol.

Alternatively, the evaluation of inhalation data and/or measurement data may be achieved by a secure data transmission via the communication device 20 to the central server that provides a feedback as to the adherence to the preset therapy protocol which is subsequently indicated at the operation unit 500. If the evaluation of the inhalation data and/or measurement data at the central server indicates, however, that the user does not follow the preset therapy protocol, then the central server may transmit a corresponding indication to control device, such as e. g. reminders to follow the preset therapy protocol, or may alter the therapy protocol to be better suitable for the user. As such, the operation device 500 may also indicate, e.g. upon advice by the physician, a change to the preset therapy protocol, for example as to changing the drug, changing the nebulization times, and the like.

In the following, an embodiment will be described in which the communication unit 100 of the control device 10 is a Bluetooth module. The present invention is, however, not limited to a Bluetooth module and other wireless communication techniques may be employed that similarly use the concept of the present invention as exemplified by the below embodiment.

The Bluetooth module is implemented into the control device 10 of the aerosol nebulizer 30 and is capable to establish a wireless Bluetooth communication connection with said communication device 20 to transfer data as to at least one of nebulization date, nebulization time, nebulization duration, a reason for a switch-off criteria, one or more drugs to be used or being used for nebulization, as well as drug combinations or the like.

Using the above Bluetooth module, the following describes an embodiment of a specific protocol or operation flow for the establishment of the communication between the control device 10 of the aerosol nebulizer 30 and a customized communication hub as an example of the communication device 20.

Based on the specific protocol a valid, reliable, and secure data transfer may be guaranteed at each time and from the beginning. In particular, it may be guaranteed that all data are correctly transferred and that, for example, inhalation data cannot get lost.

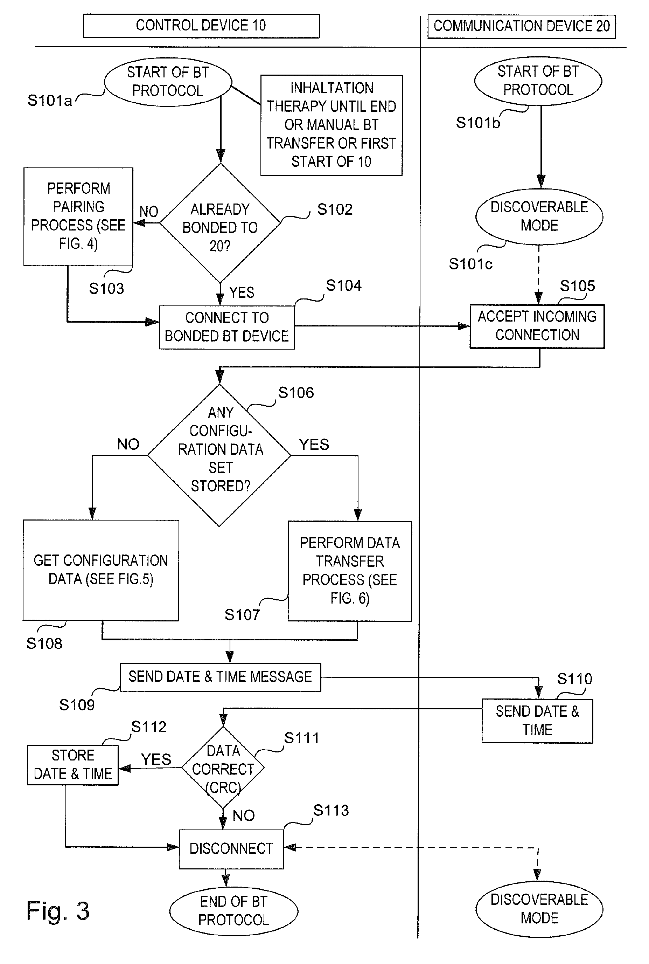

**Fig. 3** shows an overview of the specific Bluetooth (BT) protocol for the communication processes between the control device 10 and the communication device 20.

In steps S101a and S101b of **Fig. 3****,** the Bluetooth protocol may be started at the respective control device 10 and the communication hub (communication device) 20 for an interval until the end of an aerosol therapy session, or for a Bluetooth data transfer that is manually triggered by the user, or for the first start of the control device 10 after receipt by the user. Here, the control device 10 is set as a Bluetooth master device, as described above, i.e. the establishment of the wireless communication connection is initiated by the control device. On the other hand, by setting the control device 10 as the Bluetooth master device, the control device 10 is not Bluetooth discoverable. In general, if the wireless Bluetooth communication connection is lost at any time, the control device 10 will not immediately try to establish a reconnection. Instead, respective data, i.e. configuration data and/or inhalation data, will be transferred the next time the control device 10 initiates the data transfer process, for example after each aerosol therapy session or if started manually by the user.

While the communication hub (communication device) 20 is in a Bluetooth discoverable mode (step S101c), in step S102 of **Fig. 3****,** it is determined by the control device 10, whether the control device 10 is already bonded to the communication device 20. If bonded, the control device 10 and the communication device 20 are paired, but not necessarily connected yet, because the pairing may also refer to a corresponding storage setting, for example in the form of corresponding flags.

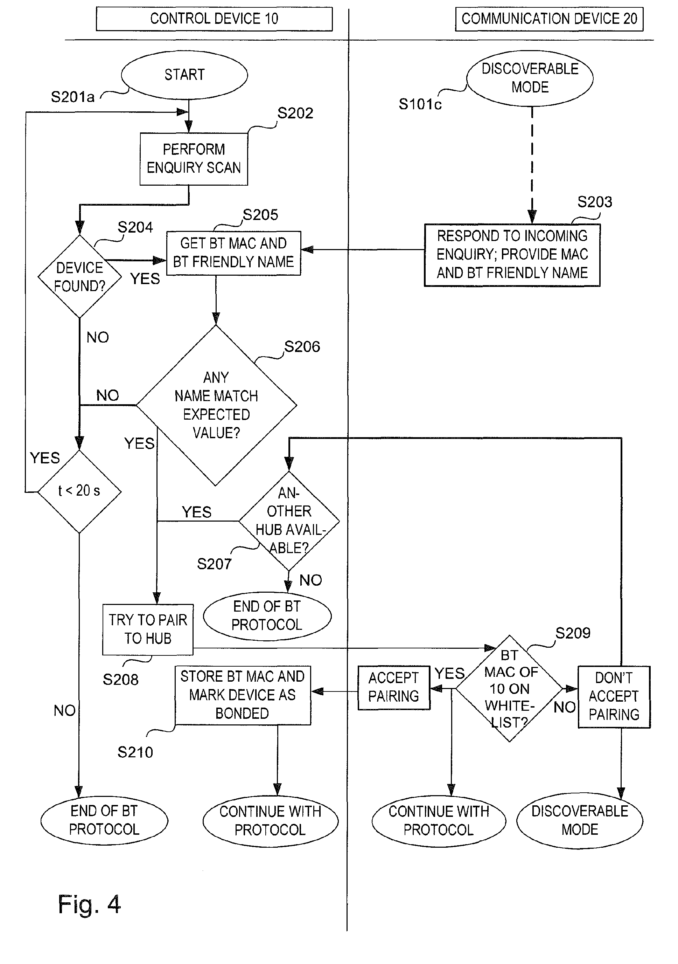

If it is determined in step S102 of **Fig. 3****,** that the control device 10 is not already bonded to the communication device 20, then the process is advanced to step S103 in which a Bluetooth pairing process is performed. This Bluetooth pairing process is further described in **Fig. 4****.** If, on the other hand, the control device 10 is already bonded to the communication device 20, then the process proceeds to step S104 in which the control device 10 attempts to connect to the bonded Bluetooth device, i.e. the communication hub (communication device) 20. Here, the control device 10 may use the communication unit 100 described above to transmit a corresponding communication message that indicates to the communication hub 20 that the control device 10 attempts to connect to the communication hub 20. Based on the established pairing, which is further described in detail in **Fig. 4****,** the communication hub 20 accepts the incoming, i.e. received connection attempt in step S105 of **Fig. 3****.**

Further, according to step S106 of **Fig. 3****,** the control device 10 determines in step S106, whether the control device 10 has stored a set of configuration data in a storage unit 300 thereof. As described above, the set of configuration data may include at least one of a current date, a current time, a maximum nebulization duration, a number of maximal storable records, an automatic data transfer deactivation, and an identification for one or more drugs usable for the liquid medication.

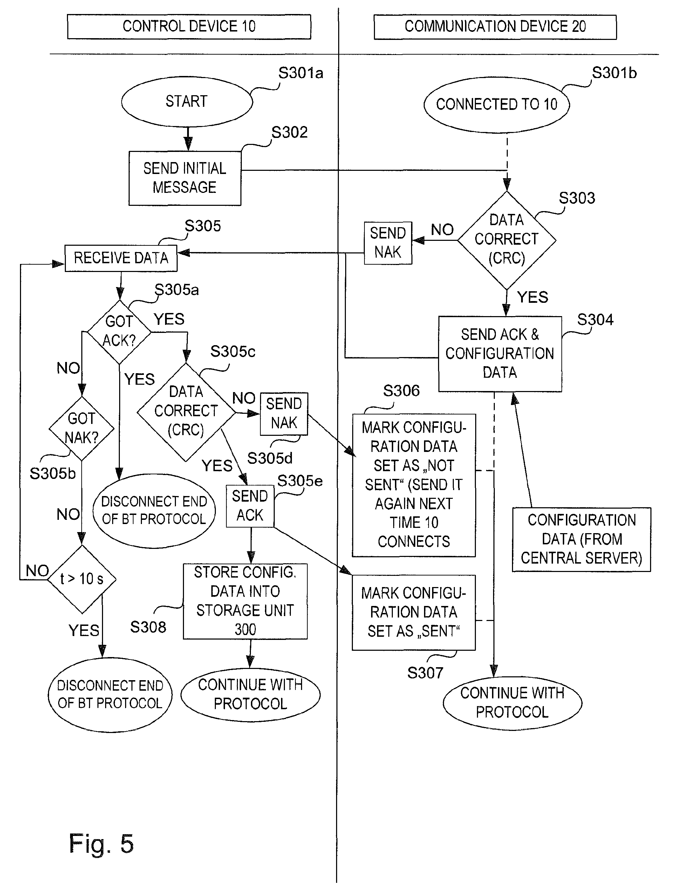

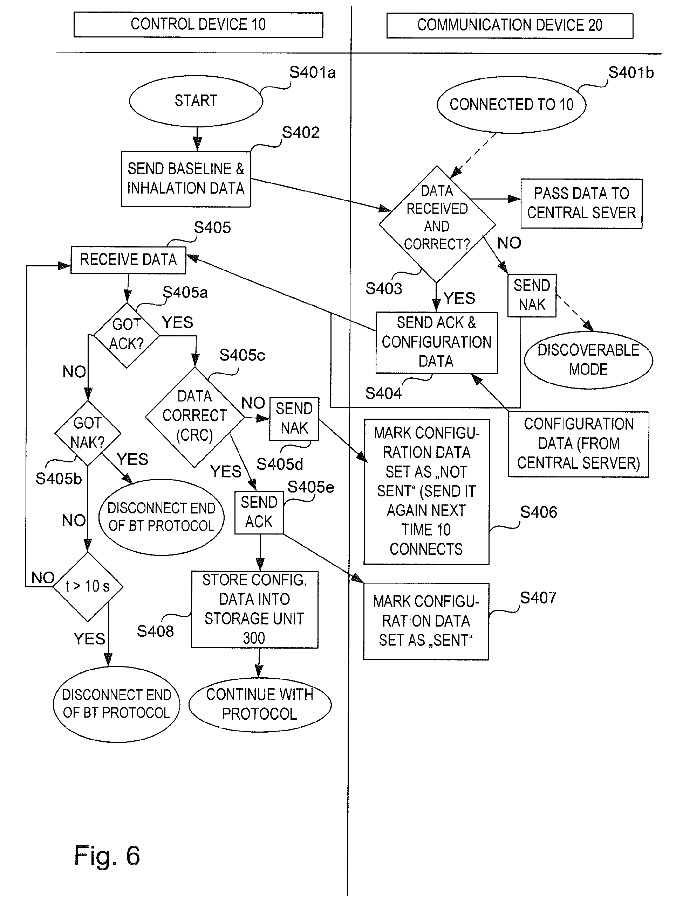

If it is determined in step S106 of **Fig. 3** that the control device 10 has stored a configuration data set, then the process proceeds with step S107 in which a data transfer process is performed which is described in detail in **Fig. 6****.** If it is determined, on the other hand, that the control device 10 has not stored a configuration data set, then the process proceeds with step S108 in which a process to receive configuration data is performed, as it is described in detail in **Fig. 5****.**

Further, in step S109 of **Fig. 3** the control device 10 may subsequently use the communication unit 100 to send a message to the communication hub (communication device) 20 to request a date and/or time value. Here, a date and/or time value may not be part of configuration data but may be transferred separately. For example, the date and time value may come from the communication hub 20, a dial-in node, or may also be generated by a central server. Responding to this message, the communication hub (communication device) 20 may send a message in step S110 that includes the requested date and/or time value (that may be local values of the communication hub (communication device) 20) together with controlling bytes, for example a check sum or hash sum to perform a cyclic redundancy check (CRC) at the control device 10 in step S111. If the transmitted check sum or hash sum corresponds to the determined check sum or hash sum, the data are determined to be correctly transmitted and the control device proceeds in step S112 by storing the date and/or time value before the wireless communication connection is disconnected in step S113 and the protocol ends. If, on the other hand, the transmitted check sum or hash sum does not correspond to the determined check sum or hash sum, the data are determined to not be correctly transmitted and the wireless communication connection is immediately disconnected in step S113 without the storing step S112.

**Fig. 4** shows an overview of the specific Bluetooth pairing process between the control device 10 and the communication device 20.
After the start of the specific Bluetooth pairing process in step S201a, which is triggered in step S103 according to **Fig. 3****,** and while the communication device 20 remains in the discoverable mode according to step S101c in **Fig. 3****,** the control device 10 performs an enquiry scan in step S202. Here, the control device 10 may perform the scan for pairable Bluetooth devices for a predetermined amount of time, for example 20 seconds.

Responding to the enquiry scan, the communication device 20 wirelessly transmits in step S203 at least one communication device identification value to the control device 10. Examples of such identification values are the Bluetooth media access control (MAC) address and/or the Bluetooth friendly NAME. Such a Bluetooth friendly NAME of the communication device may be set by the manufacturer of the communication device.

In step S204 of Fig. **4****,** the control device 10 determines whether any pairable Bluetooth devices are found within the predetermined amount of time. If no pairable Bluetooth device is found within the predetermined amount of time, then the process according to **Fig. 4** and the overall Bluetooth protocol ends. On the other hand, for each pairable Bluetooth device that is found in step S204, the control device 10 will get the at least one transmitted communication device identification value in step S205, and will compare the transmitted identification value (here, for example, the MAC address and/or the Bluetooth friendly NAME) with internally stored identification values in step S206. Identification values may be stored in advance, i.e. during the manufacturing process, in the storage unit 300 of the control device. That means that the control device 10 is provided during the manufacturing process with an identification of a communication hub (communication device) 20 that is allowed to be paired with the control device 10. For example, the control device 10 will compare the Bluetooth friendly NAME with an internal Bluetooth friendly NAME stored in advance. If there is a match, the control device 10 will try to pair with the communication hub (communication device) 20 in step S208 and send an initial message to the communication hub (communication device) 20 in order to get configuration data for the control device 10. In addition, if more than one matching communication hub (communication device) 20 is found in step S207, then the control device 10 may try to establish a Bluetooth pairing with the communication hub (communication device) 20 that is found at first, or alternatively is first in a stored list.

In step S208, when trying to pair with the communication hub (communication device) 20, the control device 10 sends a control device identification value to the communication hub (communication device) 20. For example, the control device 10 may send a control device MAC address.

In step S209, the communication hub 20 determines whether the control device MAC address sent in step S208 is stored in the communication hub 20 in association with the MAC address of the communication hub 20. In particular, the communication hub 20 is provided in advance, with a whitelist that includes at least one allowable combination/association of a control device identification value and a communication hub (communication device) identification value. For example, the whitelist includes an allowable combination of the MAC addresses of the control device 10 and the communication hub

(communication device) 20. This whitelist is stored in a storage unit of the communication hub (communication device) 20 in advance to indicate an allowable pairing even before the first actual communication process between the control device 10 and the communication hub (communication device) 20 occurs.

If the transmitted control device MAC address is on the whitelist in association/combination with the MAC address of the communication hub 20, i.e. if the pairing is allowed from the beginning, then the pairing is accepted and the control device 10 receives a pairing accepted message from the communication hub (communication device) 20. Accordingly, the process is followed by step S210 in which the control device 10 stores the MAC address of the communication hub (communication device) 20, and marks the communication hub (communication device) 20 as bonded. Accordingly, the overall Bluetooth protocol is continued according to step S103 in **Fig. 3****.** If, on the other hand, the transmitted control device MAC address is not on the whitelist in association/combination with the MAC address of the communication hub 20, i.e. if the pairing is not allowed from the beginning, then the pairing is not accepted and the communication hub (communication device) 20 may not respond to the pairing accepted message and instead remains in the discoverable mode.

**Fig. 5** illustrates a flow diagram of a configuration data transfer process between the control device 10 and the communication hub (communication device) 20. As described above, this process is triggered according to the overall Bluetooth protocol in step S108 of **Fig. 3****.** This configuration data transfer process is performed because the control device needs a configuration data set to operate, and therefore needs the configuration data set before the first usage. It is to be noted that the configuration data set may be changed, and thus may be updated during the lifetime of the control device 10 and/or the aerosol nebulizer 30, for example when the aerosol therapy protocol is changed. According to step S301a and S301b of **Fig. 5** the configuration data transfer process starts off while the communication hub (communication device) 20 is wirelessly connected to the control device 10.

In step S302 of **Fig. 5****,** the control device 10 may use the communication unit 100 to send an initial message to the communication hub (communication device) 20 in order to request a transmission of configuration data. Subsequently, the communication hub (communication device) 20 may perform a cyclic redundancy check (CRC) on the received data (here, for example, the connection message including a serial number, a CRC, or the like) to determine whether the data are correct.

If the data are determined to not be correct, then the communication hub (communication device) 20 may subsequently send a negative acknowledgment (NAK) protocol message to the control device.

If, on the other hand, the data are determined to be correct, then the communication hub (communication device) 20 may transmit in step S304 of **Fig. 5** both a (positive) acknowledgement (ACK) protocol message and the requested set of configuration data to the control device 10. Here, the set of configuration data may be provided to the communication hub (communication device) 20 from a central server. In a preferred embodiment, this central server may be configured to check whether previous configuration data were correctly delivered to the control device 10, before providing a new or updated set of configuration data. If the previous set of configuration data was not delivered correctly to the control device 10, then this previous set of configuration data is cancelled before providing the new or updated set of configuration data to the communication hub (communication device) 20.

In step S305 of **Fig. 5****,** the control device 10 may receive the data sent in step S304, and may determine whether the received data include an ACK protocol message (step S305a). If this is not the case and it is determined in S305b that the received data include a NAK protocol message, then the wireless communication connection is disconnected and the protocol ends. Further, in the case that the received data neither include an ACK protocol message nor a NAK protocol message within a predetermined amount of time, for example 10 seconds, then also in this case the wireless communication connection is disconnected and the protocol ends.

If, on the other hand, the received data include an ACK protocol message, then the control device 10 determines in step 305c whether the received data are correct. For example, the control device 10 may be configured to perform a cyclic redundancy check (CRC) on the data received in step S305.

When the control device 10 determines the correctness of the received data and determines, for example based on a calculated checksum or hash sum over the received data, that the data are incorrect, then the communication unit 100 of the control device 10 may transmit in step S305d a corresponding NAK protocol message to the communication hub (communication device) 20. Accordingly, the communication hub (communication device) 20 marks the set of configuration data as "not sent", for example by using a corresponding flag, and attempts to send it again the next time when the control device 10 establishes a connection. If, as described above, a central server in the meantime provides a new or updated set of configuration data, then the communication hub (communication device) 20 cancels or deletes the marked set of configuration data before sending the new set of configuration data.

In step S305e, the control device 10 acknowledges the reception of the configuration data set if the cyclic redundancy check (CRC) is valid, i.e. the checksum or hash sum is received correctly. Accordingly, the communication hub (communication device) 20 marks the set of configuration data as "sent" (step S307).

Further, in step S308 of **Fig. 5** the control device 10 may store the received set of configuration data in the storage unit 300, for example in an internal EEPROM or the like.

**Fig. 6** illustrates a flow diagram of a data transfer process between the control device 10 and the communication hub (communication device) 20. As described above, this process is triggered according to the overall Bluetooth protocol in step S107 of **Fig. 3****.** According to step S401a and S401b of **Fig. 6** the data transfer process starts off while the communication hub (communication device) 20 is wirelessly connected to the control device 10.

In step S402 of **Fig. 6** the control device 10 may encrypt the inhalation data and/or additional baseline data before sending it to the communication hub (communication device) 20. In addition, the control device 10 may also encode, for example BASE64 encode the inhalation data and/or the additional baseline data. Subsequently, the control device 10 may use the communication unit 100 to transmit the encrypted inhalation data and/or additional baseline data to the communication hub (communication device) 20. It is to be noted that the communication hub (communication device) 20 does not decrypt or decode the received inhalation data and/or baseline data, and instead transmits the encrypted inhalation data and/or baseline data to the central server. Instead, the communication hub (communication device) 20 only determines in step S403, based on the enclosed controlling bytes and performing a CRC check, for example, as described in step S303 of Fig. 5, whether the data are correctly received.

The remaining steps in **Fig. 6** (S404 - S408) are similar to the corresponding steps S304 - S308 in **Fig. 5** and a corresponding description is omitted here. In particular, the remaining steps in **Fig. 6** (S404 - S408) are performed if new configuration data are available that should be transferred. If no new configuration data are available, then a corresponding message (for example comprising a NULL) is transmitted.
Based on the established wireless Bluetooth communication connection such data may be transferred from the control device 10 to the communication device 20, and further to a central server which may preferably be performed via a cloud server. On this basis, tele-monitoring features may be implemented for the aerosol nebulizer in a secure and reliable way, and thus allow adherence monitoring for example within clinical trials of inhaled drug products.

An example of an appropriate system (indicated by dashed lines in Fig. 2) includes, in a patient environment, the control device 10 and the aerosol nebulizer 30. This system may also include the communication device 20. The control device 10 establishes a wireless Bluetooth communication connection with the communication device 20 which is a communication hub in this example. Data, as described above, may be encrypted in the control device 10 and are subsequently transferred from the control device 10 to the communication hub 20 via said Bluetooth connection. The communication hub may establish a communication connection with a cloud server, for example using radio communication, the internet, or the like, to transfer the encrypted data and identification values of both the control device 10 and the communication hub 20 to the cloud server. The cloud server may perform a relaying of the transferred data and identification values to a central mapping server, or alternatively directly to a corresponding customer server. The relaying between the cloud server and the mapping server and/or the central server may be performed via the internet or the like. Here, using the transferred identification values the central mapping server may perform a mapping to transfer the data to an appropriate customer server that applies the adherence monitoring and adherence control. For example, data from control devices 10 of aerosol nebulizers 30 within a clinical trial are duly sent to a separate clinical trial/study server where the data are stored. Further, the separate clinical trial/study server may implement software/hardware tools for data display, data evaluation, report generation, and/or feedback control to the control devices 10. In this example, data decryption may be performed by either the central mapping server or the customer server.

## Claims

1. Control device (10) for an aerosol nebulizer comprising an aerosol generator for nebulizing a liquid medication, said control device (10) comprising:
a communication unit (100), configured to establish a wireless communication connection and to perform data communication with a communication device (20);
a control unit (200), configured to control an operation of the aerosol generator;
a storage unit (300), configured to store in advance an identification value of said communication device (20), wherein, based on said identification value, said communication unit (100) is further configured to establish said wireless communication connection by an authentication of the communication device (20) based on the identification value of the communication device (20).

2. Control device according to claim 1, wherein said communication unit (100) is further configured to authenticate the communication device (20) by comparing said stored identification value of said communication device (20) with a received identification value of said communication device (20) which is received from said communication device (20) when establishing said wireless communication connection for a first time.

3. Control device according to claim 2, wherein said communication unit (100) is further configured to initiate a pairing process to establish said wireless communication connection by transmitting a pairing message including a control device (10) identification value to said communication device (20), if said stored identification value matches said received identification value.

4. Control device according to claim 3, wherein said communication unit (100) is further configured to receive a pairing acceptance message from the communication device (20), if said transmitted control device (10) identification value matches a control device (10) identification value stored in advance in association with a communication device (20) identification.

5. Control device according to one of claims 1 - 4, wherein said communication unit (100) is further configured to receive configuration data for operating the aerosol nebulizer from the communication device (20).

6. Control device according to claim 5, wherein said configuration data comprise at least one of a current date, a current time, a maximum nebulization duration, a number of maximal storable records, automatic data transfer deactivation, and one or more drugs usable for said liquid medication.

7. Control device according to one of claims 5 - 6, wherein said communication unit (100) is further configured to determine whether the configuration data are correctly transmitted from the communication device (20).

8. Control device according to claim 7, wherein said communication unit (100) is further configured to send an acknowledgement message to the communication device (20), if the configuration data are correctly transmitted.

9. Control device according to one of claims 1 - 8, wherein the communication unit (100) is further configured to enclose inhalation data by controlling bytes.

10. Control device according to one of claims 1 - 8, wherein the data communication includes inhalation data of at least one of a nebulization date, a nebulization time, a nebulization duration, a communication time of the communication device (20), a reason for a switch-off of the aerosol nebulizer, usage status by a user, fill level of the liquid medication in the reservoir, inhalation/exhalation flow/volume/time characteristics of a user, and a list of one or more drugs to be used or being used for said liquid medication.

11. Control device according to one of claims 1 - 10, further including:
an encryption unit (400), configured to encrypt data used for the data communication with the communication device (20).

12. Control device according to one of claims 1 - 11, wherein the control device is set as a master device when establishing said wireless communication connection with said communication device (20).

13. Control device according to one of claims 1 - 12, wherein the communication unit (100) is further configured to not initiate re-establishment of said wireless communication connection with said communication device (20), if said wireless connection is lost.

14. Control device according to one of claims 1 - 13, wherein said communication unit (100) is further configured to transfer said inhalation data at an end of a therapy session to the communication device (20).

15. Control device according to one of claims 1 - 14, wherein said communication unit (100) is further configured to deactivate said transfer of inhalation data via the communication unit (100) at an end of a therapy session to the communication device (20).

16. Control device according to one of claims 1 - 14, wherein said communication unit (100) is further configured to receive an acknowledgement message from the communication device (20), if said inhalation data are correctly transmitted to said communication device (20).

17. Control device according to one of claims 1 - 16, further comprising:
an operation unit (500), by which a user is able to select and/or confirm a liquid drug to be used in said aerosol nebulizer for nebulizing said liquid medication.

18. Control device according to one of claims 1 - 16, further comprising:
an operation unit (500), by which a user is able to select an operation mode of said control device.

19. Control device according to one of claims 1 - 16, further comprising:
an operation unit (500), by which a user is able to monitor a drug-specific adherence to a preset therapy protocol.

20. Control device according to one of claims 1 - 19, wherein the communication device (20) is a Bluetooth communication device and said wireless communication connection is a Bluetooth connection.

21. A system, comprising:
said control device (10) according to any of claims 1 - 20, and
said aerosol nebulizer.
